# EUROPÄISCHE PATENTSCHRIFT

(11) **EP 0 539 811 B1**
(45) Veröffentlichungstag und Bekanntmachung des Hinweises auf die Patenterteilung: **03.04.1996**
(21) Anmeldenummer: 92117719.2
(22) Anmeldetag: 16.10.1992
(51) Int. Cl.: C07C 279/26, A61K 31/155, A61K 7/18, A61K 7/22

(54) **Chlorhexidin-Addukt**
Chlorhexidine adduct
Produit d'addition de la chlorhexidine

(30) Priorität: 26.10.1991 DE 4135397
(43) Veröffentlichungstag der Anmeldung: 05.05.1993
(73) Patentinhaber: IVOCLAR AG, FL-9494 Schaan (LI)
(72) Erfinder: Rheinberger, Volker, Dr., FL-9490 Vaduz (LI); Burtscher, Peter, Dr., A-6714 Nütziders (AT); Salz, Ulrich, Dr., W-8995 Weissenberg (DE)
(74) Vertreter: UEXKÜLL & STOLBERG

(56) Entgegenhaltungen:
- EP-A- 0 110 568
- DE-A- 2 430 280
- GB-A- 1 369 942
- CHEMICAL ABSTRACTS, vol. 105 Columbus, Ohio, US; abstract no. 146198, HUANG, XIN ET AL. 'Prevention of dental caries by chlorhexidine dihydrofluoride. I. The influence of chlorhexidine dihydrofluoride on the surface properties of hydroxylapatide and the dissolution rate of calcium from hydroxylapatite'
- CHEMICAL ABSTRACTS, vol. 105 Columbus, Ohio, US; abstract no. 146197, WANG, QIN ET AL. 'The caries preventive effect of chlorhexidine hydrofluoride. II. The effect of chlorhexidine hydrofluoride at low concentration in acid gel on the demineralization of enamel'

## Beschreibung

Die Erfindung betrifft ein Chlorhexidin-Addukt, das als Antiseptikum und insbesondere als Antiseptikum in der Zahnheilkunde sowie als therapeutisches und prophylaktisches Antiplaquemittel eingesetzt werden kann.

Bei dem Versuch, die Bildung von Plaque und damit auch von Karies zu inhibieren bzw. vollständig zu unterbinden, sind in der Vergangenheit Substanzen mit antibakteriellen Eigenschaften wie z.B. chlorierte Phenole, Formaldehyd und quartäre Ammoniumverbindungen auf ihre Wirksamkeit hin überprüft worden. Diese haben aufgrund ihrer Toxizität und ihres eingeschränkten Wirkungsspektrums jedoch keinen Eingang in die Praxis gefunden.

Das derzeit wirksamste Antiplaquemittel ist Chlorhexidin (1,6-bis-(N⁵-p-Chlorphenyl-N'-diguanido)-hexan), welches insbesondere in Form des wasserlöslichen Digluconats, aber auch als schwerlösliches Diacetat und Dihydrochlorid verwendet wird (vgl. hierzu, A. Scheie in J. Dent. Res. 68, 1609 (1989) und P. Gjermo in J. Dent. Res. 68, 1602 (1989)). Neben diesen Chlorhexidinverbindungen ist auch Chlorhexidin-Dihydrofluorid bekannt, welches gemäß DE-OS 21 58 150 als antiseptisches Mittel in transparenten Zahngelen Verwendung findet.

Es ist gezeigt worden, daß mit der Verwendung von Chlorhexidin als Chemotherapeutikum Bakterien vom Typ Streptococcus mutans wirksam begegnet werden kann. Bakterien dieses Typs spielen bei der Bildung von Karies eine wesentliche Rolle. Daher wird angenommen, daß mit der Verringerung ihrer Menge auf der Zahnoberfläche der Bildung von Karies wirksam entgegengetreten werden kann (vgl. hierzu I. Ostela und J. Tenovuo in Scand. J. Dent. Res. 98, 1 (1990)).

Die bakterizide Wirkung, die Chlorhexidin gegenüber Bakterien des Typs Streptococcus mutans ausübt, ist jedoch stark abgeschwächt, wennn es in geringen Konzentrationen eingesetzt wird. Daher ist auch Chlorhexidin in der praktischen Anwendung deutlichen Einschränkungen unterworfen, wenn es darum geht, Zahnplaque zu verringern, die ihrerseits zur Entstehung von Parodontose und Karies führen kann. Darüber hinaus kann die Anwendung von Chlorhexidin in höheren Konzentrationen zu unerwünschten Verfärbungen der Zunge, Zähne, Prothesen und Füllungen führen (vgl. hierzu L. Flötra, P. Gjermo, G. Rölla und J. Waerhaug in Scand. J. Dent. Res. 79, 119 (1971)).

Die GB-PS 1 369 942 offenbart schließlich wasserunlösliche polymere Salze des 1,6-Di-(p-chlorophenyl-biguanido)hexans, welche sich als Bestandteile von Zusammensetzungen für die Oralhygiene eignen. Diese Salze enthalten 1,6-Di-(p-chlorophenyl-biguanido)hexan-Dikationen sowie Anionen mit mehr als einer negativen Ladung und führen auch bei wiederholter Anwendung nicht zu einer Verfärbung des Zahnschmelzes.

Der Erfindung liegt daher die Aufgabe zugrunde, ein Chlorhexidin-Addukt zur Verfügung zu stellen, das allgemein als Antiseptikum einsetzbar ist und insbesondere als Antiplaquemittel verwendet werden kann, wobei es selbst in sehr geringen Konzentrationen wirksam der Neubildung und dem Wachstum von Zahnbelägen entgegenwirkt und darüber hinaus durch Abgabe von Fluorid in der Lage ist, den Zahnschmelz vor einer Demineralisierung insbesondere durch Säuren zu schützen.

Diese Aufgabe wird durch das neue Chlorhexidin-Addukt gemäß Anspruch 1 und das Verfahren zu seiner Herstellung gemäß der Ansprüche 2 bis 6 sowie seine Verwendung gemäß den Ansprüchen 7 und 8 gelöst.

Bei dem erfindungsgemäßen Chlorhexidin-Addukt handelt es sich um eine Verbindung mit folgender Formel: 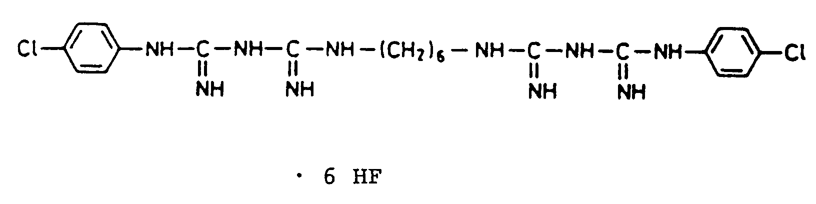
oder dessen Hydrate.

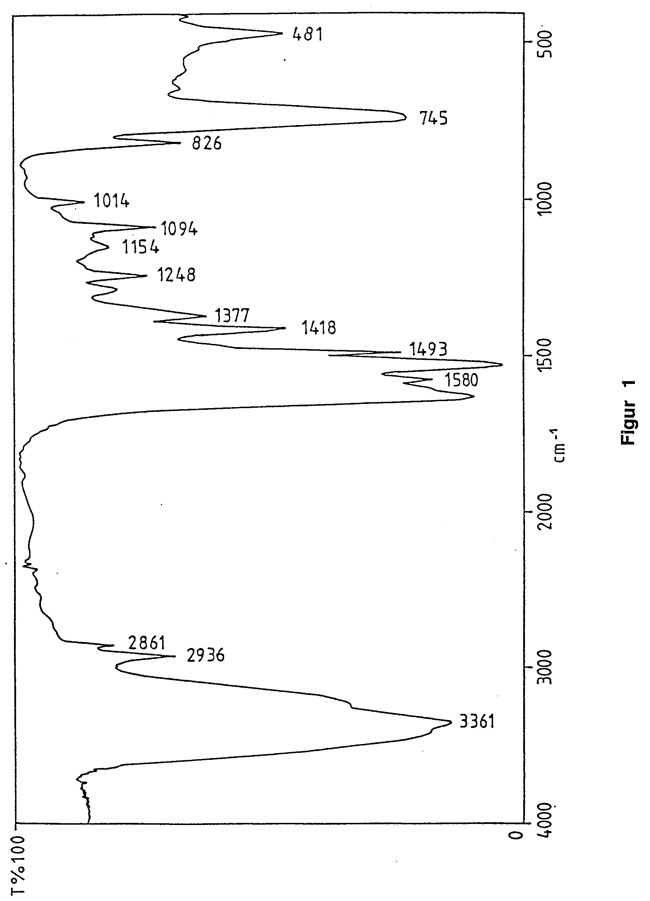

Das Addukt weist das IR-Spektrum gemäß Figur 1 auf. In welcher Weise die sechs Moleküle Fluorwasserstoff in dem erfindungsgemäßen Addukt an das Chlorhexidin-Molekül gebunden sind, ist nicht bekannt. Grundsätzlich ist vorstellbar, daß das erfindungsgemäße Addukt aus elektrisch neutralen Molekülen besteht oder in Form von Ionen und damit als Salz vorliegt.

Das erfindungsgemäße Addukt wird hergestellt, indem eine Lösung von Fluorwasserstoff mit einer Lösung von Chlorhexidinsalz bei einem Molverhältnis von Fluorwasserstoff zu Chlorhexidinsalz von mindestens 6 : 1 umgesetzt und das entstandene Addukt abgetrennt wird. Wenn ein Molverhältnis von Fluorwasserstoff zu Chlorhexidinsalz von weniger als 6 : 1 angewendet wird, so erhält man zwar ebenfalls das erfindungsgemäße Addukt, jedoch in einer geringeren Ausbeute.

Bevorzugt wird das erfindungsgemäße Chlorhexidin-Addukt dadurch hergestellt, daß eine Lösung von Fluorwasserstoff in Wasser mit einer Lösung von Chlorhexidinsalz in Wasser in einem Molverhältnis von 6 : 1 bis 30 : 1 von Fluorwasserstoff zu Chlorhexidinsalz bei einer Temperatur im Bereich von Raumtemperatur bis Rückflußtemperatur umgesetzt und der entstandene Niederschlag abgetrennt wird.

Zur Erzielung von hohen Ausbeuten und Herstellung eines leicht durch Filtration abtrennbaren Niederschlages des erfindungsgemäßen Chlorhexidin-Adduktes ist ein Molverhältnis von 10 : 1 bis 20 : 1 von Fluorwasserstoff zu Chlorhexidinsalz besonders bevorzugt. Vorteilhafterweise werden anstelle der wäßrigen Lösungen der Addukte Lösungen in einem Gemisch aus Ethanol/Wasser 90/10-Vol.% bei Rückflußtemperatur eingesetzt. In diesem Fall ist lediglich ein Molverhältnis von 8:1 von Fluorwasserstoff zu Chlorhexidinsalz erforderlich. Das erfindungemäße Addukt fällt dabei in einer hohen Ausbeute von mehr als 95% an. Aufgrund des geringen Bedarfes an Fluorwasserstoff ist diese Variante des Herstellungsverfahrens besonders bevorzugt.

Zur Herstellung des erfindungsgemäßen Adduktes wird besonders bevorzugt Chlorhexidindigluconat eingesetzt. Es können aber auch andere in dem jeweils verwendeten Lösungsmittel ausreichend lösliche Chlorhexidinsalze, wie z.B. das Dihydrochlorid oder das Diacetat Verwendung finden.

Als Reaktionsdauer sind typischerweise 24 Stunden ausreichend, um zu einer vollständigen Reaktion zu kommen. Abhängig von den gewählten Reaktionsparametern kann die Dauer der Reaktion jedoch variieren. Die für den jeweiligen Fall am besten geeignete Reaktionsdauer kann jedoch in einfacher Weise durch Routineexperimente ermittelt werden.

Das bei der Reaktion überwiegend als Niederschlag anfallende Chlorhexidin-Addukt wird bevorzugt durch Filtration und anschließendes Waschen mit Wasser und Aceton abgetrennt und gereinigt. Durch Aufarbeitung der Mutterlaugen kann weiteres Chlorhexidin-Addukt gewonnen werden, so daß insgesamt Ausbeuten von 91 bis annähernd 100% erzielbar sind. Der gereinigte Feststoff wird anschließend in bekannter Weise getrocknet und liegt danach, je nach Trocknungsgrad, in Form von Hydraten mit unterschiedlichem Wassergehalt vor. Vorzugsweise wird die Trockung bei 50°C im Trockenschrank durchgeführt.

Aufgrund seiner starken antibakteriellen Wirkung kann das erfindungsgemäße Chlorhexidin-Addukt generell als antiseptisches Mittel eingesetzt werden. Dabei kann es sowohl in pharmazeutischen als auch in kosmetischen Produkten als therapeutisches und prophylaktisches Bakterizid Verwendung finden. Bevorzugt wird es jedoch in Dentalmaterialien, wie z.B. Zahnlacken, Fissurenversieglern, Prophylaxepasten, Mundwässern, Zahnstochern, Zahnseide, Zahnkaugummi, Wundverbänden, Zahnsalben, Gingiviatrainern, Desinfektionsmitteln für Prothesen und Abformmaterialien, Trocknungsmitteln, Unterfüllungsmaterialien, Zementen, Füllungsmaterialien, Haftvermittlern und Endodontiematerialien, verwendet. Dabei kann das erfindungsgemäße Addukt auf ein festes Substrat, wie z.B. Zahnstocher oder Zahnseide, aufgebracht oder in Dentalwerkstoffe, wie z.B. provisorische Füllungsmaterialien und Fissurenversiegler, eingearbeitet werden.

Besonders vorteilhaft ist die Einarbeitung des erfindungsgemäßen Adduktes in Dentalmaterialien, die über einen beschränkten Zeitraum in der Mundhöhle verbleiben sollen, wie z.B. provisorische Füllungsmaterialien, Wundverbände, Abformmaterialien und temporäre Zemente. Wird das erfindungsgemäße Addukt beispielsweise in ein provisorisches Füllungsmaterial eingearbeit, so erhält man nach dessen Entfernen eine keimfreie Kavität, in die die endgültige Füllung unmittelbar gelegt werden kann.

Da das Chlorhexidin-Addukt nur eine recht geringe Löslichkeit in gängigen Lösungsmitteln aufweist, wird es vorzugsweise als Feststoff in die genannten Dentalmaterialien eingearbeitet. Dabei wird es in Mengen von 0,1 bis 20 Gew.%, bevorzugt 1 bis 10 Gew.% und besonders bevorzugt 3 bis 7 Gew.%, bezogen auf das gesamte Gewicht des Werkstoffes, den Dentalwerkstoffen zugesetzt. Beispiele für einsetzbare Dentalwerkstoffe sind solche, die 10 bis 95 Gew.% polymerisierbares organisches Bindemittel, 5 bis 90 Gew.% anorganische und/oder organische Füllstoffe und 0,01 bis 5 Gew. % Katalysatoren, bezogen auf das Gewicht des gesamten Werkstoffes, enthalten.

Darüber hinaus können Lösungen mit einem Gehalt von 0,03 bis 0,001 Gew% an erfindungsgemäßem Addukt verwendet werden. Als Lösungsmittel sind z.B. Wasser, Ethanol, Aceton, Ethylacetat, Triethylenglykoldimethacrylat und Decandioldimethacrylat geeignet. Ferner können künstliche oder natürliche Harze verwendet werden, die in gängigen Lösungsmitteln löslich sind und nach dem Verdunsten des Lösungsmittel hart werden. Beispiele hierfür sind Schellack, Benzoinharz, Polyvinylpyrrolidon und Kolophonium.

Eine weitere bevorzugte Anwendung des Chlorhexidin-Adduktes ist diejenige als therapeutisches oder prophylaktisches Antiplaquemittel. Dabei verhindert es die Neubildung von Zahnbelägen und inhibiert das Wachstum von bereits vorhandenen Zahnbelägen. Erkrankungen, die durch das Vorhandensein von Zahnbelägen hervorgerufen werden, wie z.B. Parodontose, primärer und sekundärer Karies und Gingivitis, lassen sich daher mit dem erfindungsgemäßen Chlorhexidin-Addukt wirksam bekämpfen.

Hinsichtlich seiner bakteriziden Wirksamkeit ist das erfindungsgemäße Addukt in einer Konzentration von 0,03 Gew.% durchaus mit dem derzeit als wirksamstes Antiplaquemittel geltenden Chlorhexidin vergleichbar. Überraschenderweise wird jedoch die Wirksamkeit von Chlorhexidin deutlich übertroffen, wenn beide in Konzentrationen von kleiner oder gleich 0,01 Gew.% eingesetzt werden. In diesem Konzentrationsbereich ist das erfindungsgemäße Chlorhexidin-Addukt auch Zinndifluorid, einer Verbindung mit bekanntermaßen sehr guten bakteriziden Eigenschaften, deutlich überlegen.

Die Überlegenheit des erfindungsgemäßen Adduktes gerade in geringen Konzentrationen ist von besonderer Bedeutung für die praktische Anwendung, denn infolge des permanenten Speichelflusses in der Mundhöhle werden aufgebrachte Wirkstoffe laufend verdünnt. Ein Wirkstoff wie das erfindungsgemäße Chlorhexidin-Addukt, der auch in niedrigen Konzentrationen einen starken bakteriziden Effekt zeigt, ist daher von besonderem Vorteil.

Ein weiterer Vorteil gegenüber Chlorhexidin ist, daß bei Verwendung des erfindungsgemäßen Adduktes keine unerwünschten Nebeneffekte wie bitterer Geschmack, Verfärbungen von Zahnmaterialien und Schleimhautirritationen auftreten.

Schließlich führt der hohe Fluorgehalt des erfindungsgemäßen Adduktes dazu, daß dieses durch Fluoridierung den Zahnschmelz schütztund daher auch in dieser Hinsicht wirksam vor der Bildung von Karies schützen kann.

Die Erfindung wird in den folgenden Beispielen näher erläutert.

### Beispiel 1

Zur Herstellung des erfindungsgemäßen Chlorhexidin-Adduktes wurden zu 45 ml einer wäßrigen 4,4 %igen (0,11 Mol) HF-Lösung 42,5 ml einer wäßrigen 20 %igen (0,01 Mol) Chlorhexidindigluconat-Lösung unter Rühren innerhalb von 2 Stunden zugetropft. Die Mischung wurde über Nacht weitergerührt, und der gebildete Niederschlag wurde filtriert und dreimal mit je 50 ml Wasser und danach zweimal mit je 50 ml Aceton gewaschen. Danach wurde der erhaltene Niederschlag bei 50°C im Trockenschrank getrocknet. Das erfindungsgemäße Chlorhexidin-Addukt fiel als Feststoff in einer Ausbeute von 76% an und hatte einen Schmelzpunkt von 185 bis 190°C.

Das IR-Spektrum (KBr-Preßling) ist in Fig. 1 wiedergegeben.

Aus der Elementaranalyse ergibt sich, daß das Produkt Chlorhexidinhexahydrofluorid mit einem bis zwei Mol Kristallwasser ist.

### Struktur A:

C₂₂ H₃₀ N₁₀ Cl₂ · 6HF · H₂O MG = 642,9

### Struktur B:

C₂₂ H₃₀ N₁₀ Cl₂ · 6HF · 2H₂O MG = 660,9

| Elementaranalyse: | | | |
|---|---|---|---|
| | gefunden | theoretisch | |
| | | Struktur A | Struktur B |
| C | 41,00% | 41,06% | 39,95% |
| H | 5,15% | 5,60% | 5,45% |
| N | 21,70% | 21,78% | 21,18% |
| Cl | 10,85% | 11,02% | 10,73% |
| F | 17,55% | 17,73% | 17,25% |
| H₂O*⁾ | 3,75% | 2,80% | 5,45% |

| | | | |
|---|---|---|---|
| *⁾ H₂O-Gehalt bestimmt nach Karl Fischer | | | |

Die Löslichkeit des erfindungsgemäßen Adduktes in einigen gängigen Lösungsmitteln und Reaktivverdünnern ist in der nachstehenden Tabelle I angegeben:

**Tabelle I**

| | |
|---|---|
| Wasser (pH-Wert 2 bis 9,7) | 0,03 Gew.% |
| Ethanol | 0,005 Gew.% |
| Aceton | 0,03 Gew.% |
| Ethylacetat | 0,02 Gew.% |
| Triethykenglykoldimethacrylat (SR-205) | < 0,005 Gew.% |
| Decandioldimethacrylat (D₃MA) | < 0,005 Gew.% |

### Beispiel 2

Durch Verwendung von 90 ml einer wäßrigen 4,4 %igen (0,2 Mol) HF-Lösung und 42,5 ml einer wäßrigen 20 %igen (0,01 Mol) Chlorhexidindigluconat-Lösung wurde - bei ansonsten gleicher Reaktionsführung wie nach Beispiel 1 - ein Niederschlag erhalten, der leichter filtrierbar als der gemäß Beispiel 1 erhaltene war.

Das Chlorhexidin-Addukt fiel in einer höheren Ausbeute von 91 bis 94% an.

### Beispiel 3

Zu 200 ml (0,08 Mol) einer Lösung von Fluorwasserstoff in einem 90/10-Vol.% Gemisch aus Ethanol/Wasser wurden bei Rückflußtemperatur während 1 Stunde 42,5 ml (0,01 Mol) einer wäßrigen 20%igen Chlorhexidindigluconat-Lösung unter Rühren zugetropft, und es wurde eine weitere Stunde gerührt. Nach dem Abkühlen des Reaktionsgemisches auf Raumtemperatur wurde der entstandene Niederschlag abfiltriert und dreimal mit je 50 ml eines 90/10-Vol.% Gemisches aus Ethanol/Wasser gewaschen. Im Gegensatz zu den bei Raumtemperatur durchgeführten Herstellungsverfahren gemäß Beispiel 1 und Beispiel 2 war der entstandene Niederschlag kristallin und daher leicht abfiltrierbar. Innerhalb einer weiteren Woche fiel aus der Mutterlauge weiteres Produkt aus. Zusammen ergab sich eine Ausbeute von 98%.

Gegenüber den Verfahrensvarianten gemäß den Beispielen 1 und 2 ist bei dieser Verfahrensführung in einem Gemisch aus Ethanol/Wasser bei Rückflußtemperatur von Vorteil, daß ein besser filtrierbarer Niederschlag in sehr hoher Ausbeute anfällt und daß der Bedarf an Fluorwasserstoff erheblich geringer ist.

### Beispiel 4

Die antibakterielle Wirksamkeit des erfindungsgemäßen Chlorhexidin-Adduktes wurde im Agar-Diffusionstest mit Streptococcus mutans nachgewiesen.

Dazu wurden Kulturabschwemmungen von Streptococcus mutans in einen flüssigen Hefe-Extrakt Dextrose-Agar eingebracht. Nach Erstarren der Agarplatten wurde ein Loch von 10 mm Durchmesser ausgestochen, in das 0,1 ml der jeweils zu untersuchenden Lösung eingefüllt wurden. Bei den jeweils doppelt angesetzten Proben wurden nach 24-stündiger Bebrütung bei 37°C die Durchmesser der Hemmhöfe ausgemessen. Die Ergebnisse dieser Untersuchungen sind in der nachstehenden Tabelle II wiedergegeben.

**Tabelle II**

| **Hemmhof-Durchmesser** | | | |
|---|---|---|---|
| Konzentration | Lösung A | Lösung B | Lösung C |
| 0,03 Gew.% | 17 mm | 17 mm | 20 mm |
| 0,01 Gew.% | 13 mm | 15 mm | 11 mm |
| 0,003 Gew.% | 11 mm | 12 mm | 10 mm* |

| | | | |
|---|---|---|---|
| * Keine Wirksamkeit | | | |
| Lösung A: Wäßrige Lösung von Chlorhexidindigluconat Lösung B: Wäßrige Lösung des erfindungsgemäßen Chlorhexidin-Adduktes Lösung C: Wäßrige Lösung von Zinndifluorid | | | |

Es zeigt sich, daß im Konzentrationsbereich von 0,03 Gew.% die antibakterielle Wirksamkeit des erfindungsgemäßen Chlorhexidin-Adduktes gegenüber Streptococcus mutans mit der von Chlorhexidindigluconat vergleichbar ist, während Zinndifluorid in diesem Konzentrationsbereich noch eine stärkere Wirkung zeigt. Mit zunehmender Verdünnung fällt jedoch die Wirksamkeit bei den bekannten Verbindungen stark, im Falle des Zinndifluorids bei einer Konzentration von 0,003 Gew.% sogar soweit, daß kein antibakterieller Effekt mehr nachgewiesen werden kann. Demgegenüber ist die antibakterielle Wirksamkeit des erfindungsgemäßen Adduktes selbst bei Konzentrationen von 0,01 bis 0,003 Gew.% noch sehr hoch. Seine Überlegenheit gerade in niedrigen Konzentrationen macht es damit zu einem sehr wirksamen Antiplaquemittel.

### Beispiel 5

Ein lichthärtender Fissurenversiegler enthält folgende Bestandteile:
56,08 Gew.% Bis-Phenol A-glycidylmethacrylat (Bis-GMA)
36,1 Gew.% Triethylenglykoldimethacrylat
0,45 Gew.% Cyanoethylmethylanilin
0,25 Gew.% DL-Campherchinon
2,1 Gew.% TiO₂
0,02 Gew.% 2,6-Di-tert.-butyl-p-kresol
5,0 Gew.% Chlorhexidin-Addukt
Durch Mischen aller Komponenten wurde der lichthärtbare Fissurenversiegler erhalten. Dieser wurde mit einem Pinsel auf die Fissuren eines Molaren gepinselt und 20 sec mit dem Lichthärtungsgerät Heliolux® der Firma Vivadent/Liechtenstein gehärtet. Auf diese Weise wurden die Fissuren dauerhaft verschlossen, und man erhielt durch die Fluorid-Abgabe des in dem Versiegler eingearbeiteten Chlorhexidin-Adduktes einen ausgezeichneten Kariesschutz im Okklusalbereich.

Durch die Zumischung von 1 bis 5 Gew.% des Chlorhexidin-Adduktes zu der Fissurenversiegler-Grundrezeptur wurde keine Abnahme der Durchhärtungstiefe beobachtet, wie folgende Werte für die Vickers-Härte zeigen:

| | HV 0,5 |
|---|---|
| Fissurenversiegler ohne Chlorhexdidin-Addukt | 188 MPa |
| Fissurenversiegler + 1% Chlorhexidin-Addukt | 248 MPa |
| Fissurenversiegler + 3% Chlorhexidin-Addukt | 212 MPa |
| Fissurenversiegler + 5% Chlorhexidin-Addukt | 180 MPa |

Zum Nachweis für die Chlorhexidin- und Fluoridmigration wurden jeweils 10 Prüfkörper mit einem Durchmesser von 50 mm und einer Höhe von 0,5 mm in dest. Wasser bei 37°C gelagert. Die Fluoridionen-Konzentration wurde mittels einer Fluorelektrode bestimmt, die Chlorhexidin-Konzentration wurde UV-spektroskopisch ermittelt. Die kumulierte Fluorid- und Chlorhexidinabgabe ist in Tabelle III zusammengefaßt.

**Tabelle III**

| Migrationszeit [Tage] | Fluoridabgabe [µg/cm²] | Chlorhexidinabgabe [µg/cm²] |
|---|---|---|
| 1 | 0,95 | 3,86 |
| 2 | 1,48 | 5,56 |
| 3 | 1,91 | 6,84 |
| 4 | 2,22 | 7,58 |
| 7 | 2,91 | 9,26 |
| 10 | 3,45 | 10,30 |
| 17 | 4,22 | 11,60 |
| 24 | 4,92 | 12,30 |
| 30 | 5,58 | 13,20 |
| 44 | 6,56 | 14,40 |

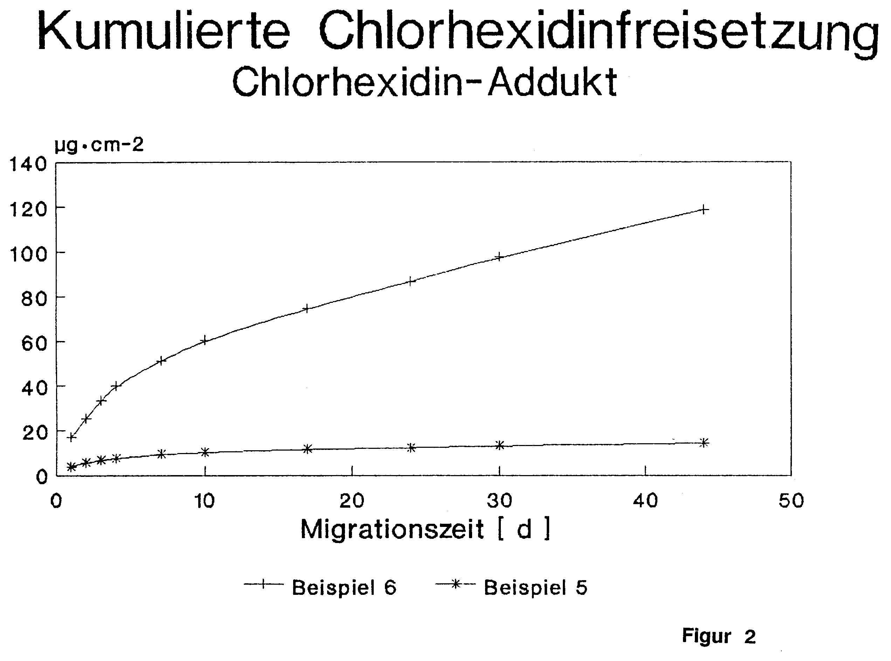

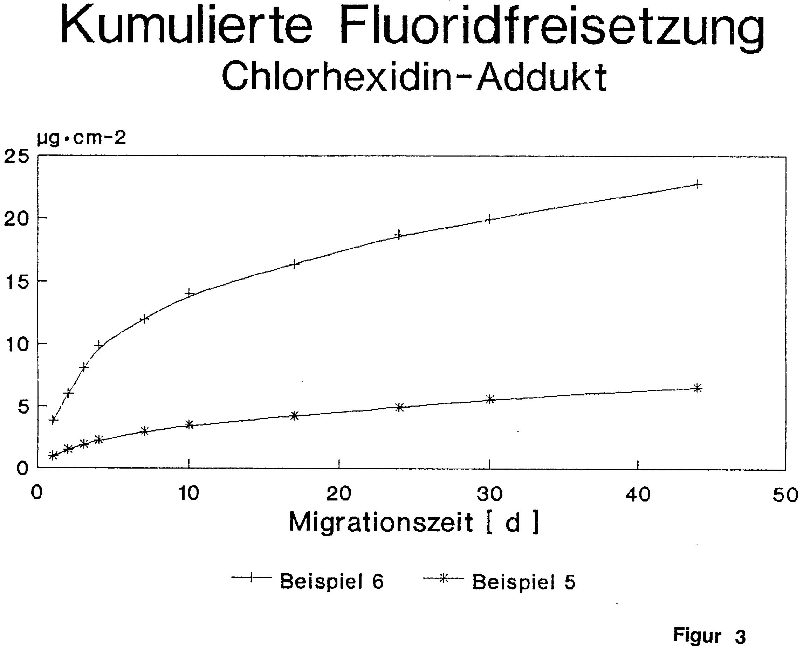

In den Figuren 2 und 3 sind die Ergebnisse graphisch dargestellt.

### Beispiel 6

Ein lichthärtender Dentalwerkstoff mit relativ hoher Wasseraufnahme und somit hoher Wirkstoff-Freisetzung (z.B. geeignet als provisorisches Füllungsmaterial oder als Wundverband) hat folgende Zusammensetzung:
43,6 Gew.% Polyesterurethandimethacrylat
0,25 Gew% Cyanoethylmethylanilin
0,15 Gew.% DL-Campherchinon
35,0 Gew.% Splitterpolymerisat
21,0 Gew.% amorphes SiO₂ silanisiert (BET-Oberfläche 50 m²/g)
Das Splitterpolymerisat besteht aus:
59,4% Urethandimethacrylat
40 % Feinteiliges SiO₂ silanisiert
0,6% Benzpinakol.

Die Komponenten werden zusammengemischt und bei 120°C polymerisiert. Das gefüllte Polymerisat wird zu einem Polymerpulver gemahlen.

Bei dem amorphen feinteiligen silanisierten SiO₂ handelt es sich um Aerosil® OX 50 der Degussa AG.

Durch Mischen aller Komponenten wurde ein lichthärtendes Dentalmaterial erhalten.

Die Wasseraufnahme von Dentalfüllungscomposites liegt normalerweise im Bereich von 1 Gew.%, dieses Material zeigt eine Wasseraufnahme im Bereich von 3 Gew.% (3 Wochen H₂O-Lagerung bei 37°C). Die kumulierte Fluorid- und Chlorhexidinabgabe ist in Tabelle IV zusammengefaßt.

**Tabelle IV**

| Migrationszeit [Tage] | Fluoridabgabe [µg/cm²] | Chlorhexidinabgabe [µg/cm²] |
|---|---|---|
| 1 | 3,78 | 17,0 |
| 2 | 6,03 | 25,7 |
| 3 | 8,03 | 33,6 |
| 4 | 9,82 | 40,3 |
| 7 | 11,98 | 51,4 |
| 10 | 13,99 | 60,6 |
| 17 | 16,37 | 74,5 |
| 24 | 18,68 | 86,6 |
| 30 | 19,88 | 97,8 |
| 44 | 22,68 | 118,3 |

In den Figuren 2 und 3 sind die Ergebnisse graphisch dargestellt.

Wie die Migrationsversuche zeigen, werden signifikante Mengen an Fluorid und Chlorhexidin aus diesem Dentalmaterial freigesetzt, so daß auch in dieser Kombination eine ausreichende Hemmung des Wachstums von Mikroorganismen zu erwarten ist.

Da nicht alle Mikroorganismen auf freigesetzte Wirkstoffe gleich reagieren, wurden Unterschungen unter Verwendung folgender Mikroben durchgeführt.
- Grampositive Bakterien:: Streptococcus mutans
Staphylococcus aureus
- Gramnegative Bakterien:: Pseudomonas auruginosa
Escherichia coli
- Pilz:: Candida albicans
Prüfkörper (d = 10 mm, h = 2 mm) wurden in die feuchten Mikroorganismen-Kulturen bei 37°C über eine Zeit von 24 Stunden eingelegt und anschließend wurde der Hemmhof bestimmt.

| | Hemmhof-Durchmesser [mm] |
|---|---|
| Streptococcus mutans | 15 |
| Staphylococcus aureus | 16 |
| Pseudomonas auruginosa | 17 |
| Escherichia coli | 15 |
| Candida albicans | 12 |

Es ist eine deutliche Wachstumshemmung bei diesen unterschiedlichen Mikroorganismen feststellbar.

## Patentansprüche

1. Chlorhexidin-Addukt der folgenden Formel 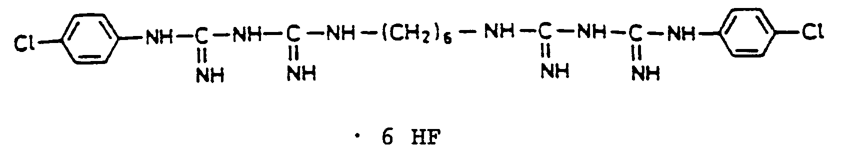 und dessen Hydrate.

2. Verfahren zur Herstellung des Chlorhexidin-Adduktes nach Anspruch 1, **dadurch gekennzeichnet**, daß
a) eine Lösung von Fluorwasserstoff mit einer Lösung eines Chlorhexidinsalzes
b) bei einem Molverhältnis von mindestens 6:1 von Fluorwasserstoff zu Chlorhexidinsalz umgesetzt und
c) der entstandene Niederschlag abgetrennt wird.

3. Verfahren nach Anspruch 2, **dadurch gekennzeichnet**, daß
a) wäßrige Lösungen eingesetzt werden,
b) das Molverhältnis von Fluorwasserstoff zu Chlorhexidinsalz 6 : 1 bis 30 : 1 beträgt und
c) die Umsetzung bei einer Temperatur im Bereich von 20°C bis Rückflußtemperatur durchgeführt wird.

4. Verfahren nach Anspruch 2, **dadurch gekennzeichnet**,
a) Lösungen in einem Gemisch aus Ethanol und Wasser eingesetzt werden,
b) das Molverhältnis von Fluorwasserstoff zu Chlorhexidinsalz mindestens 8 : 1 beträgt und
c) die Umsetzung bei Rückflußtemperatur durchgeführt wird.

5. Verfahren nach Anspruch 4, dadurch gekennzeichnet, daß in Schritt a) ein 90/10-Vol.% Gemisch aus Ethanol und Wasser eingesetzt wird.

6. Verfahren nach einem der Ansprüche 2 bis 4, **dadurch gekennzeichnet**, daß Chlorhexidindigluconat als Chlorhexidinsalz eingesetzt wird.

7. Verwendung eines Chlorhexidin-Addukts oder seines Hydrats nach Anpruch 1 zur Herstellung eines Antiseptikums.

8. Chlorhexidin-Addukt oder sein Hydrat nach Anspruch 1 zur Verwendung als Mittel zur Verhütung von Karies.

9. Verwendung eines Chlorhexidin-Addukts oder seines Hydrats nach Anpruch 1 zur Herstellung eines Mittels zur Verhütung von Karies.

10. Dentalmaterial, **dadurch gekennzeichnet**, daß es das Chlorhexidin-Addukt oder sein Hydrat nach Anspruch 1 enthält.

## Claims

1. Chlorhexidine adduct of the following formula: 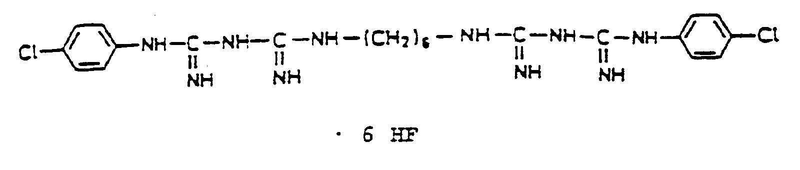 and hydrates thereof.

2. Process for the preparation of the chlorhexidine adduct according to Claim 1, characterized in that
a) a solution of hydrogen fluoride is reacted with a solution of a chlorhexidine salt
b) at a molar ratio of hydrogen fluoride to chlorhexidine salt of at least 6:1 and
c) the precipitate formed is separated off.

3. Process according to Claim 2, characterized in that
a) aqueous solutions are employed,
b) the molar ratio of hydrogen fluoride to chlorhexidine salt is 6 : 1 to 30 : 1 and
c) the reaction is carried out at a temperature in the range from 20°C to the reflux temperature.

4. Process according to Claim 2, characterized in that
a) solutions in a mixture of ethanol and water are employed,
b) the molar ratio of hydrogen chloride to chlorhexidine salt is at least 8 : 1 and
c) the reaction is carried out at the reflux temperature.

5. Process according to Claim 4, characterized in that a 90/10% by volume mixture of ethanol and water is employed in set a).

6. Process according to one of Claims 2 to 4, characterized in that chlorhexidine digluconate is employed as the chlorhexidine salt.

7. Use of a chlorhexidine adduct or of its hydrate according to Claim 1 for the preparation of an antiseptic.

8. Chlorhexidine adduct or its hydrate according to Claim 1 for use as an agent for prevention of caries.

9. Use of a chlorhexidine adduct or of its hydrate according to Claim 1 for the preparation of an agent for the prevention of caries.

10. Dental material, characterized in that it contains the chlorhexidine adduct or its hydrate according to Claim 1.

## Revendications

1. Adduit de chlorhexidine de formule suivante 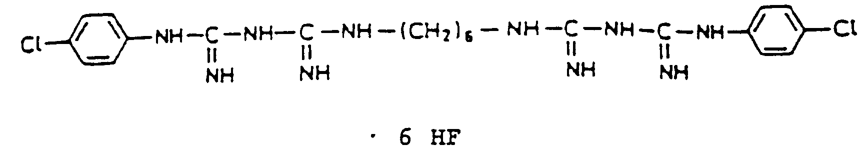 et ses hydrates.

2. Procédé pour la préparation de l'adduit de chlorhexidine selon la revendication 1, caractérisé en ce que
a) on fait réagir une solution d'acide fluorhydrique avec une solution d'un sel de chlorhexidine,
b) à un rapport molaire de l'acide fluorhydrique au sel de chlorhexidine d'au moins 6:1, et
c) on sépare le précipité résultant.

3. Procédé selon la revendication 2, caractérisé en ce que
a) on utilise des solutions aqueuses,
b) le rapport molaire de l'acide fluorhydrique au sel de chlorhexidine va de 6:1 à 30:1, et
c) on effectue la réaction à une température dans la plage allant de 20°C à la température de reflux.

4. Procédé selon la revendication 2, caractérisé en ce que
a) on utilise des solutions dans un mélange d'éthanol et d'eau,
b) le rapport molaire de l'acide fluorhydrique au sel de chlorhexidine est d'au moins 8:1, et
c) on effectue la réaction à la température de reflux.

5. Procédé selon la revendication 4, caractérisé en ce que, dans l'étape a), on utilise un mélange d'éthanol et d'eau à 90:10 % en volume.

6. Procédé selon l'une des revendications 2 à 4, caractérisé en ce que, comme sel de chlorhexidine, on utilise le digluconate de chlorhexidine.

7. Utilisation d'un adduit de chlorhexidine, ou de son hydrate selon la revendication 1, pour la préparation d'un antiseptique.

8. Adduit de chlorhexidine, ou son hydrate selon la revendication 1, pour utilisation en tant qu'agent pour la prévention de caries.

9. Utilisation d'un adduit de chlorhexidine, ou de son hydrate selon la revendication 1, pour la préparation d'un agent destiné à la prévention de caries.

10. Matériau dentaire, caractérisé en ce qu'il contient l'adduit de chlorhexidine ou son hydrate selon la revendication 1.
